# EUROPEAN PATENT APPLICATION

(11) **EP 3 647 431 A1**
(43) Date of publication of application: **06.05.2020**
(21) Application number: 17916084.1
(22) Date of filing: 27.06.2017
(51) Int. Cl.: C12P 19/30, C12N 1/06, C12N 1/16

(54) **METHOD FOR PRODUCING A YEAST-BASED PRODUCT WITH HIGH NUCLEOTIDE CONCENTRATION**

(71) Applicant: Fertinagro Biotech, S.L., 44195 Teruel (ES)
(72) Inventor: ATARES REAL, Sergio, 44195 Teruel (ES); ROMERO LOPEZ, Joaquin, 44195 Teruel (ES); SALAET MADORRAN, Ignasi, 44195 Teruel (ES); FERRER GINES, María, 44195 Teruel (ES); NARANJO OLIVERO, Miguel Angel, 44195 Teruel (ES); YANCE CHAVEZ, Tula del Carmen, 44195 Teruel (ES); ALIGUE ALEMANY, Rosa, 44195 Teruel (ES)
(74) Representative: González López-Menchero, Álvaro Luis
(86) International application number: PCT/ES2017/070463
(87) International publication number: WO 2019/002634

(57) **Abstract**

The present invention relates a method for producing a yeast-based product with a high nucleotide concentration, specifically with a nucleotide content of more than 80% by weight. The method is essentially based on carrying out a first enzymatic digestion with pepsin in the presence of iron ions and a second enzymatic digestion with a Fe chelating agent, using a starting product rich in nucleotides, such as a yeast, thereby obtaining a yeast-based end-product with a high nucleotide concentration, specifically higher than 80% by weight.

## Description

The present invention relates to a method for producing a yeast-based product with a high nucleotide concentration, specifically with a nucleotide content of more than 80% by weight.

The method of the invention is essentially based on carrying out a first enzymatic digestion with pepsin in the presence of iron ions and a second enzymatic digestion with a Fe chelating agent, using a starting product rich in nucleotides, such as a yeast, thereby obtaining a yeast-based end-product with a high nucleotide concentration, specifically higher than 80% by weight.

The objective of intensive agriculture techniques is to increase crop yields and they are based on the use of materials such as chemical fertilisers, pesticides and herbicides, which are known to cause environmental damage and pose a risk to human health. In order to reduce the use of chemical fertilisers, for example, products are available on the market which are labeled as "organic" due to their biological origin, such as composts, nitrogen-fixing microorganisms, phosphorus and potassium-solubilising microorganisms, etc.

Furthermore, the preparation and application of inoculants based on different microbial species (biofertilisers) is a well-known practice in agriculture. Thus, the use of yeasts as biofertilisers is of great interest, since they are a rich source of vitamins, especially B-complex vitamins, constitute a natural source of amino acids and proteins and are also a very rich source of iron (see for example, "Vegetative growth, chemical composition, and flavonoids content of Azadirachta indica plants as affected by application of yeast natural extract", Lobna S. Taha et al., Journal of Applied Pharmaceutical Science Vol. 6 (04), pp. 093-097, April, 2016; Mady, M.A., "Effect of foliar application with yeast extract and zinc on fruit setting and yield of faba bean (Vicia faba L), J. Biol. Chem. Environ. Sci., 2009, Vol. 4(2): 109-127). Furthermore, certain yeasts synthesise anti-microbial substances, vegetative growth factors and cell division-stimulating cytokines (Barnet et al., 1990; El-Desouky et al. 1998; Ahmad et al. 2014). Yeasts represent an abundant and reliable source of bioactive compounds that provide properties such as stimulation of vegetative growth and/or protection against different stresses, both biotic and abiotic (Hanafy et al., "Effect of Some Natural Extracts on Growth and Chemical Constituents of Schefflera arboricola Plants", Journal of Horticultural Science & Ornamental Plants 4 (1): 26-33, 2012; Hammad & Ali 2014; Abdelhamid et al. 2014; Kowalczy K & Zielony T 2008; Mohamed 2006; Shalaby & El-Ramady 2014), in the context of sustainable agriculture.

The use of yeast extracts and yeasts is already known in the fertiliser industry. In this respect, see for example document CN 1966663 ("Composite microorganism foliage fertilizer bacteria agent and its producing method and use"), which describes a microbial agent composed of Saccharomyces cerevisiae, Lactobacillus plantarum, Mucor racemosus and Aspergillus oryzae, and which is used as a foliar fertiliser to stimulate the growth of deep-rooting and foliage plants on improving cultivated soils and increasing crop yield.

Yeast cells have a chemical composition of approximately 40% of proteins, 15% of nucleic acids, 25% of polysaccharides, 15% of lipids and 5% of hydrosoluble compounds such as nucleotides, amino acids, sugars, growth factors and enzymes, inter alia (PEREZ, M., (2000), "Obtainment of a hydrolysate from distillery yeast cream and evaluation of its prebiotic activity", Agrarian University of Havana, Cuba, 2000).

One of the cellular components of the yeasts of special interest are nucleotides. Given the importance of these components in agriculture, providing a method that will make it possible to obtain a product rich in nucleotides from raw materials rich in these components and readily available, and especially from yeasts, would be desirable and constitutes an object of the present invention.

Document ES 2 004 241, for example, describes a method for preparing an extract of the yeast *Candida utilis* CS 7529 (FERM BP-1656) or *Candida utilis* CSB 6316 (FERM BP-1657), containing 5'-IMP (inosine-5'-monophosphate), 5'GMP (guanosine-5'-monophosphate), natural or similar, in a high concentration, from an extract containing RNA obtained from RNA-rich viable yeast cells, at temperatures between 80°C and 120°C, obtaining a RNA-containing extract and subsequently treating the extract with phosphodiesterase-5. The yeast extract thus obtained has an improved flavour.

In accordance with the previously described object, the invention relates to a method for producing a nucleotide-rich product from yeasts. The method is essentially based on carrying out a first enzymatic digestion with pepsin in the presence of iron ions and a second enzymatic digestion with a Fe chelating agent, using a starting product rich in nucleotides, cell bodies of a yeast, thereby obtaining a yeast-based end-product with a high nucleotide concentration, specifically higher than 80% by weight.

In the present description, the concepts "yeast" and "yeast cell bodies" are used as synonyms, unless specified otherwise.
Figure 1 shows the result of electrophoresis in 0.8% agarose gel, validating the digestion of various DNAs and RNAs by a commercial porcine pepsin. Lane 1, 2: Salmon sperm DNA; Lane 3, 4: λDNA; Lane 5, 6: pET-28a; Lane 7, 8: M13mp18; Lane 9, 10: RNA staircase. Other conditions: 4.0 mg/ml of pepsin, NaH₂PO₄ buffer (25 mM, pH 3.8, including 200 mM NaCl), 37°C, 5 h. For the RNA, digestion time was 1 hour (Source: Liu, Y., et al. (2015), "Digestion of Nucleic Acids Starts in the Stomach". Scientific Reports, 5, 11936);
Figure 2 shows the result of the electrophoresis of a plasmid DNA (pDNA) using different concentrations of iron ions and sodium and calcium chloride (control) as a comparison, to validate the different concentrations of iron ions (Fe²⁺) capable of inhibiting the asDNA activity of the pepsin in comparison. Buffer solution, pH 2.5, 5 h, at 37°C.
Figure 3 shows the result of the electrophoresis of bodies of yeasts and not plasmid DNA using different concentrations of iron ions and sodium and calcium chloride (control) as a comparison, demonstrating the greater inhibition by the iron ions a) and maintaining its protease activity b).
Figure 4 shows the chelating effect on the iron ions of the EDTA (ethylenediaminetetraacetic acid) on pDNA.
Figure 5 shows the chelating effect on the iron ions of the EDTA (ethylenediaminetetraacetic acid) on bodies of yeasts.

Specifically, the method of the invention includes the following stages:
a. A first enzymatic digestion of yeast cell bodies carried out by pepsin in the presence of Fe²⁺/Fe³⁺ ions, as an inhibiting agent of the asDNA activity of the pepsin, to achieve a selective digestion of the proteins, maintaining the DNA of said cell bodies undigested;
b. Separation of the soluble parts from the insoluble parts, wherein the DNA is found, by means of filtration or centrifugation;
c. A second enzymatic digestion carried out through the incorporation of a Fe chelating agent in order to prevent the Fe²⁺/Fe³⁺ ion from inhibiting the action of the pepsin on the DNA; and
d. Separation of the insoluble parts from the soluble parts, wherein the nucleotides are found.

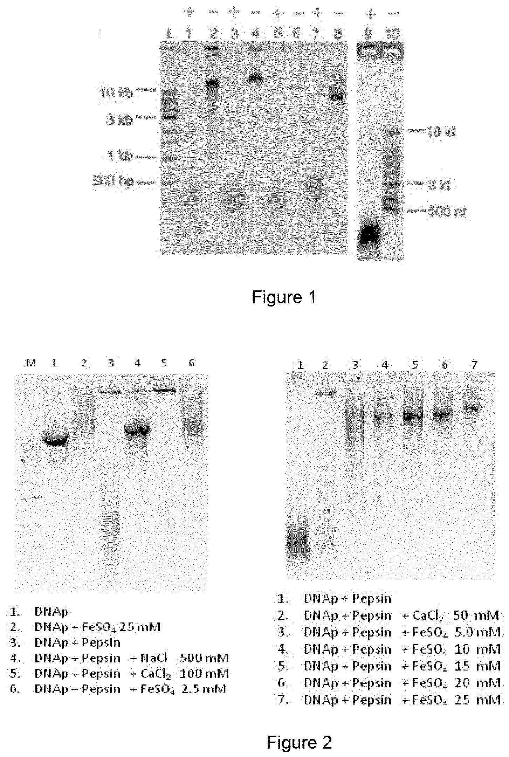

The use of pepsin in the first enzymatic digestion of the method derives from its capacity to digest both proteins and DNA (Liu, Y., et al. (2015), "Digestion of Nucleic Acids Starts in the Stomach". Scientific Reports, 5, 11936; Zhang Y, et al. (2016), "The effects of food components on the digestion of DNA by pepsin", International Journal of Food Sciences and Nutrition 7, 67, 2016, see Figure 1). This capacity of the pepsin over the DNA is inhibited by adding certain salts such as NaCl, KCI, MgCl₂ and CaCl₂ to the medium in concentrations ranging between 100 and 500 mM.

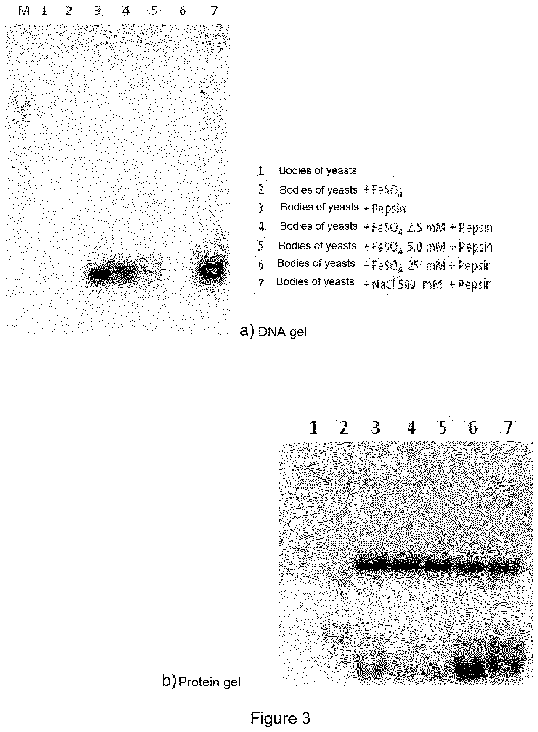

As indicated above, in stage a) of the method of the invention, yeast cell bodies are firstly digested in the presence of a concentration of Fe²⁺/Fe³⁺ ions in order to inhibit the capacity of the pepsin to digest the DNA (asDNA capacity). In the present invention, the use of the Fe²⁺/Fe³⁺ ion as an inhibitor of the digestive capacity of the pepsin over the DNA is justified by its capacity up to 100 times more powerful than any salt described to date (NaCl, CaCl₂, see Figure 3).

Next, the insoluble parts containing DNA, which remains intact in the insoluble parts, are separated in stage b).

The following stage c) is the incorporation of a Fe²⁺/Fe³⁺ chelating agent; in this manner the Fe²⁺/Fe³⁺ ions are no longer available and the inhibiting action over the pepsin cannot be carried out, due to which it can begin to digest the DNA.

In stage d), the soluble parts are separated from the insoluble parts, wherein the nucleotides are found in the soluble parts in this stage.

The method makes it possible to produce a product rich in nucleotides which, in an additional stage e) of the method, are dried by means of atomisation or freeze-drying to obtain a dry product with a minimum nucleotide content of 80%.

In one embodiment of the method of the invention, the first enzymatic digestion of stage a) takes place in a buffer solution at pH 2.5, at 37°C, for approximately 16 hours. The buffer solution to be used may be, for example, a 25 mM glycine buffer.

Although the yeast to be used in the present invention is not particularly limited, in a preferred embodiment cell bodies of *Saccharomyces cerevisiae* are used.

The source of Fe²⁺/Fe³⁺ ions is not particularly limited, and inorganic or organic iron salts such as iron sulfate (II), iron chloride (III), ferric ammonium citrate or ferrous gluconate may be used as a source of Fe²⁺/Fe³⁺ ions. Preferably, in the process of the invention, particularly in the first enzymatic digestion of stage a), Fe²⁺ is used as an iron ion in the form of ferrous sulfate. Preferably, the concentration of ferrous sulfate or Fe²⁺ ions ranges between 2.5 and 50 mM.

In one embodiment, the second enzymatic digestion of stage c) takes place in a buffer solution at pH 2.5, at 37°C, for approximately 24 hours, in order to achieve the maximum digestion until obtaining nucleotides.

Although in the present invention any iron chelating agent can be used, the chelating agent is preferably selected from ethylenediaminetetraacetic acid (EDTA), hydroxyethylethylenediaminetriacetic acid (HEDTA), diethylenetriaminepentaacetic acid (DTPA), ethylenediamine-N,N'-di[(orthohydroxyphenylacetic)] acid (o,o-EDDHA), ethylenediamine-di-(o-hydroxymethylphenylacetic) acid (o,o-EDDHMA), ethylenediamine-di-2-hydroxy-4-carboxyphenylacetic acid (EDDCHA) and ethylenediamine-N,N-bis(2-hydroxy-5-sulfo)phenylacetic acid (EDDHSA). With special preference, EDTA is used as a chelating agent in stage c).

Preferably, the chelating agent cited in stage c) is used in a chelating agent: Fe²⁺/Fe³⁺ ion ratio of 0.1:5.

With respect to additional stage d), the drying of the nucleotide-rich product (content greater than 80%) is carried out by atomisation or freeze-drying.

Next, the invention is explained based on the following examples of the invention, examples which are illustrative and non-limiting thereof, and in reference to the figures.

### Example 1: Determination of the inhibiting dose of Fe over the asDNA activity of the pepsin

In order to determine the minimum concentration capable of inhibiting the asDNA activity of the pepsin, different concentrations of Fe²⁺/Fe³⁺ were assayed in a plasmid DNA (pDNA). To this end, an assay was conducted using 1 µg of purified pDNA with different concentrations of FeSO₄, at concentrations of 25, 20, 15, 10, 5.0 and 2.5 mM, in a buffer solution at pH 2.5, for 5 h, at 37°C.

NaCl and CaCl₂ salts were previously used as a control. The results of the electrophoresis are shown in Figure 2, wherein it can be observed that the iron is capable of inhibiting the asDNA capacity of the pepsin, even at concentrations of 2.5 mM.

### Example 2: Effect of the presence of Fe on the Protease and asDNA activities of the pepsin using yeast extract as a source of raw material to be digested

The same digestion conditions as in Example 1 were assayed, but using bodies of yeasts and not pure pDNA as material to be digested. The results of the electrophoresis are shown in Figure 3, wherein the inhibition of the asDNA capacity of the pepsin can be observed, but maintaining its protease activity. As shown in the figure, there is partial inhibition of the asDNA activity at a concentration of iron ions of 2.5 mM; at 5.0 mM this inhibition increases, although not completely. Complete inhibition of the digestion of the cell DNA is obtained at a concentration of iron ions of 25 mM, these inhibition results being better than in the previously described salts.

### Example 3: The incorporation of a Fe chelating agent reestablishes the asDNA activity of the pepsin

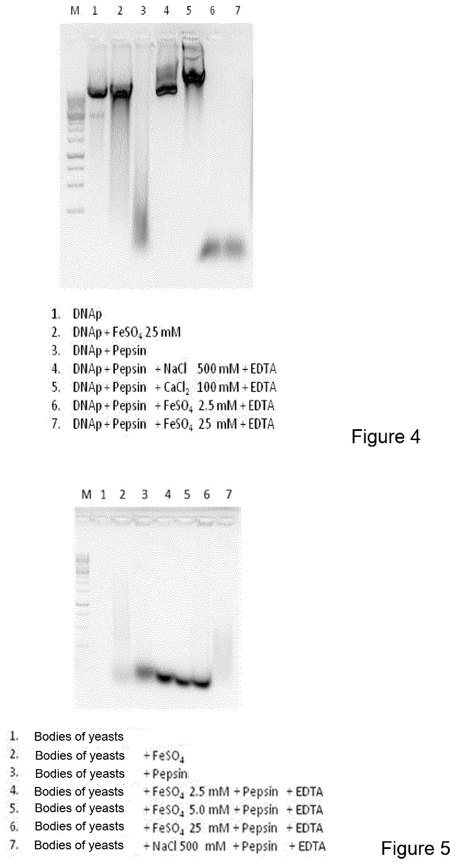

In order to demonstrate that the sequestration of the soluble iron with the incorporation of a chelating agent reestablishes the asDNA activity of the pepsin, an assay was conducted using 1 µg of purified pDNA with concentrations of FeSO₄ of 2.5 mM and 25 mM in a buffer solution at pH 2.5, using a chelating agent, in this case EDTA, at a concentration of 3X the concentration of iron ions. The reaction was carried out for 5 h at 37°C. The previously described salts were used as a control. The results of the electrophoresis are shown in Figure 4. In accordance with the results, it can be observed that the concentration of the chelating agent EDTA is sufficient to sequester the Na⁺ or Ca²⁺, probably due to their high concentrations. However, this chelating agent is capable of sequestering the Fe, enabling the maximum digestion of the pDNA, both at concentrations of 2.5 mM and 25 mM.

### Example 4: The incorporation of a Fe chelating agent reestablishes the asDNA activity of the pepsin using yeast extract as a source of raw material to be digested

The same protocol as in Example 3 is carried out, but using bodies of yeasts and not pDNA as a source of raw material to be digested. The results are shown in Figure 5. As can be observed, at a higher concentration of Fe/chelate there is maximum digestion of nucleic acids.

### Example 5: Embodiment of the method of the invention using a concentration of 25 mM Fe (II), using iron sulfate (II) as a salt and 50 mM of EDTA as a chelating agent

Protein digestion of an amount of 500 g of *Saccharomyces cerevisiae* yeasts was carried out under the following conditions: buffer tampon at pH 2.5, FeSO₄ 25 mM, for 16 hours at 37°C and 4 µg/ml of pepsin. The mixture was centrifuged at 8,000 g to obtain the insoluble part wherein the DNA is found. This fraction is subsequently used for the second digestion.

A buffer solution at pH 2.5 was added in the second digestion, the chelating agent EDTA (50 mM) was added in a concentration of 2X and supplemented with pepsin until obtaining a final concentration of 4 µg/ml, and the digestion was carried out for 24 hours at 37°C.

Upon completion of the digestion, tangential filtering was carried out with a pore size of 1 KDa to obtain the soluble part, wherein the nucleotides are found. Lastly, the permeate of the previous stage was atomised.
Process yield: 41.23 grams of product.
Nucleotide purity: 91.1 %

### Example 6: Embodiment of the method of the invention using a concentration of 5 mM of Fe (III) using iron chloride (III) as a salt and 15 mM of EDTA as a chelating agent

Protein digestion of an amount of 500 g of *Saccharomyces cerevisiae* yeasts was carried out under the following conditions: buffer tampon at pH 2.5, FeSO₄ 5 mM, for 16 hours at 37°C and 4 µg/ml of pepsin. The mixture was centrifuged at 8,000 g to obtain the insoluble part wherein the DNA is found. This fraction is subsequently used for the second digestion.

A buffer solution at pH 2.5 was added in the second digestion, the chelating agent EDTA (50 mM) was added in a concentration of 3X and supplemented with pepsin until obtaining a final concentration of 4 µg/ml, and the digestion was carried out for 24 hours at 37°C.

Upon completion of the digestion, tangential filtering was carried out with a pore size of 1 KDa to obtain the soluble part, wherein the nucleotides are found. Lastly, the permeate of the previous stage was atomised.
Process yield: 52.23 grams of product.
Nucleotide purity: 84.1%

### Example 7: Embodiment of the method of the invention using a concentration of 25 mM Fe (II), using iron sulfate (II) as a salt and 5 mM of o,o-EDDHA as a chelating agent

Protein digestion of an amount of 500 g of *Saccharomyces cerevisiae* yeasts was carried out under the following conditions: buffer tampon at pH 2.5, FeSO₄ 5 mM, for 16 hours at 37°C and 4 µg/ml of pepsin. The mixture was centrifuged at 8,000 g to obtain the insoluble part wherein the DNA is found. This fraction is subsequently used for the second digestion. The mixture was centrifuged at 8,000 g to obtain the insoluble part wherein the DNA is found. This fraction is subsequently used for the second digestion.

A buffer solution at pH 2.5 was added in the second digestion, the chelating agent o,o-EDDHA (5 mM) was added in a concentration of 0.2X and supplemented with pepsin until obtaining a final concentration of 4 µg/ml, and the digestion was carried out for 24 hours at 37°C.

Upon completion of the digestion, tangential filtering was carried out with a pore size of 1 KDa to obtain the soluble part, wherein the nucleotides are found. Lastly, the permeate of the previous stage was atomised.
Process yield: 53.23 grams of product.
Nucleotide purity: 92.02%.

## Claims

1. A method for producing a product with a high nucleotide concentration, specifically with a nucleotide content of more than 80% by weight, from a yeast, wherein the method comprises the stages of
a. A first enzymatic digestion of yeast cell bodies carried out by pepsin in the presence of Fe²⁺/Fe³⁺ ions, as an inhibiting agent of the asDNA activity of the pepsin, to achieve a selective digestion of the proteins, maintaining the DNA of said cell bodies undigested;
b. Separation of the soluble parts from the insoluble parts, wherein the DNA is found, by means of filtration or centrifugation;
c. A second enzymatic digestion carried out through the incorporation of a Fe chelating agent in order to prevent the Fe²⁺/Fe³⁺ ion from inhibiting the action of the pepsin on the DNA; and
d. Separation of the insoluble parts from the soluble parts, wherein the nucleotides are found.

2. The method for producing a product with a high nucleotide concentration, according to claim 1, **characterised in that** it also includes an additional drying stage e) by means of atomisation or freeze-drying to obtain a dry product having a minimum nucleotide content of 80%.

3. The method for producing a product with a high nucleotide concentration, according to claim 1 or 2, **characterised in that** the Fe²⁺/Fe³⁺ ions come from an organic or inorganic iron salt selected from the group consisting of iron sulfate (II), iron chloride (III), ferric ammonium citrate or ferrous gluconate.

4. The method for producing a product with a high nucleotide concentration, according to claim 3, **characterised in that** the Fe²⁺/Fe³⁺ ions come from iron sulfate (II).

5. The method for producing a product with a high nucleotide concentration, according to claim 4, **characterised in that** the concentration of iron sulfate (II) ranges between 2.5 and 50 mM.

6. The method for producing a product with a high nucleotide concentration, according to claim 1 or 2, **characterised in that** the Fe²⁺/Fe³⁺ chelating agent is used in a chelating agent: Fe²⁺/Fe³⁺ ion ratio of 0.1:5.

7. The method for producing a product with a high nucleotide concentration, according to claim 1 or 2, **characterised in that** the Fe²⁺/Fe³⁺ chelating agent is selected from ethylenediaminetetraacetic acid (EDTA), hydroxyethylethylenediaminetriacetic acid (HEDTA), diethylenetriaminepentaacetic acid (DTPA), ethylenediamine-N,N'-di[(orthohydroxyphenylacetic)] acid (o,o-EDDHA), ethylenediamine-di-(o-hydroxymethylphenylacetic) acid (o,o-EDDHMA), ethylenediamine-di-2-hydroxy-4-carboxyphenyl-acetic acid (EDDCHA) and ethylenediamine-N,N-bis(2-hydroxy-5-sulfo)phenylacetic acid (EDDHSA).

8. The method for producing a product with a high nucleotide concentration, according to claim 6, **characterised in that** the chelating agent is EDTA.

9. The method for producing a product with a high nucleotide concentration, according to claim 1 or 2, **characterised in that** the first enzymatic digestion of stage a) takes place in a buffer solution at pH 2.5, at 37°C, for approximately 16 hours.

10. The method for producing a product with a high nucleotide concentration, according to claim 1 or 2, **characterised in that** the second enzymatic digestion of stage c) is carried out in a buffer solution at pH 2.5, at 37°C, for approximately 24 hours.

11. The method for producing a product with a high nucleotide concentration, according to any of the preceding claims, **characterised in that** the yeast to be used consists of cell bodies of *Saccharomyces cerevisiae.*

12. Use of soluble iron (II) or iron (III) salts for inhibiting the asDNA activity of the pepsin in a method for obtaining nucleotides from a yeast in order to produce a nucleotide-rich product, with a content greater than 80%.
